(19) Europäisches Patentamt
European Patent Office
Office européen des brevets

(11) Publication number: **0 398 617 A2**

(12) **EUROPEAN PATENT APPLICATION**

(21) Application number: **90305182.9**

(22) Date of filing: **14.05.90**

(51) Int. Cl.[5]: **C07B 57/00, C07D 451/02, C07D 453/02, C07D 471/08, C07D 487/08, C07F 5/02, //(C07D471/08,223:00,221:00), (C07D487/08,209:00,209:00)**

(30) Priority: **15.05.89 GB 8911081**
**12.10.89 GB 8923014**

(43) Date of publication of application:
**22.11.90 Bulletin 90/47**

(84) Designated Contracting States:
**CH DE FR GB IT LI NL**

(71) Applicant: **MERCK SHARP & DOHME LTD.**
**Hertford Road**
**Hoddesdon Hertfordshire EN11 9NU(GB)**

(72) Inventor: **Showell, Graham Andrew**
**5 Beehive Lane**
**Welwyn Garden City, Hertfordshire(GB)**
Inventor: **Snow, Roger John**
**29 East Gate Road**
**Danbury, Connecticut 06810(US)**

(74) Representative: **Barrett-Major, Julie Diane et al**
**Merck & Co., Inc. European Patent Department Terlings Park Eastwick Road**
**Harlow Essex CM20 2QR(GB)**

(54) **Process for resolving 1-azabicyclo [2.2.1]heptane-3-carboxylates.**

(57) A process for preparing a substantially pure enantiomer of a 1-azabicyclo[2.2.1]heptane, 1-azabicyclo[3.2.1] octane or quinuclidine, substituted on the carbon atom $\beta$ to the ring nitrogen by a carboxy group, by synthesising and separating diastereomeric derivatives thereof wherein the hydroxy group of the carboxy is replaced by a residue of a chiral amine or $\alpha$-amino acid.

EP 0 398 617 A2

## PROCESS FOR RESOLVING 1-AZABICYCLO[2.2.1]HEPTANE-3-CARBOXYLATES

The present invention relates to a process for resolving enantiomers of compounds useful as intermediates in the synthesis of oxadiazoles having muscarinic agonist activity.

In European published patent specifications nos. 239309, 257741 and 261763 are disclosed certain azabicyclic compounds, including oxadiazole derivatives thereof, having, for example, muscarinic agonist activity and processes for their preparation. The processes disclosed are multi-step and include for example those which proceed via intermediates of formula (A)

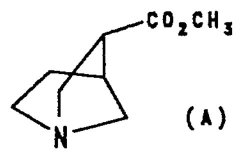

(A)

including analogues and derivatives thereof. Both the final azabicyclic compounds and the intermediates of formula (A) have at least one asymmetric centre and can therefore exist as both enantiomers and diastereomers. Some, such as the intermediates of formula (A), can exist as exo- and endo- isomers. However, no process is disclosed wherein the optical isomers of the final azabicyclic compounds (nor the intermediates of formula (A) nor its analogues and derivatives) can separately be prepared nor the racemic mixture resolved.

Thus, in order to prepare individual enantiomers of the oxadiazoles mentioned above and other substituted azabicycles, attempts were made to resolve optically active intermediates used in their preparation. Various of the conventional methods were tried, but without complete success. For example, it was found that using chiral acids such as tartaric and camphor-10-sulphonic was unsuccessful for preparing substantially pure enantiomers of, for example, 1-azabicyclo[2.2.1]heptanes and 1-azabicyclo[3.2.1]octanes. Likewise, the use of chiral esters such as derivatives of menthol, N-benzoyl-2-amino-1-butanol and N-benzoyl norephedrine did not work as either they could not be prepared or the chiral derivatives of the azabicycles would not separate. It was then surprising to find that a chiral amide of an intermediate could be prepared and could give rise to separation of the enantiomers, as desired.

Hence, the present invention provides a process for preparing a substantially pure enantiomer of a 1-azabicyclo[2.2.1]heptane, 1-azabicyclo[3.2.1] octane or quinuclidine, substituted on the carbon atom $\beta$ to the ring nitrogen by a carboxy group, which process comprises:

(a) synthesising diastereomeric derivatives thereof wherein the hydroxy group of the carboxy is replaced by a residue of a chiral amine or $\alpha$-amino acid;

(b) separating said diastereomeric derivatives thereof to give a substantially pure diastereomer of said derivative; and

(c) hydrolysing the substantially pure diastereomer of said derivative so produced to give the corresponding substantially pure enantiomer.

This method has the advantages of forming a stable chiral intermediate which is separable by crystallisation or chromatography into the constituent enantiomers.

There is therefore provided a process for preparing substantially pure enantiomers of formula (I)

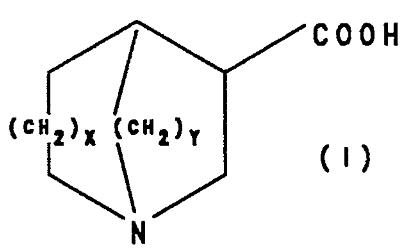

(I)

wherein x is 0 or 1, y is 1 or 2 and x plus y is less than 3, in exo-, endo- or a mixture of exo- and endo-forms; or a reactive derivative thereof, which process comprises

(a) synthesising diastereomers of formula (II):

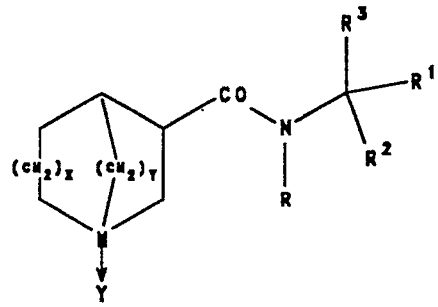

(II)

wherein R, $R^1$, $R^2$ and $R^3$ are each selected from H, alkyl, aryl and aralkyl, and $R^1$ is additionally selected from a carboxylic acid derivative, provided that $R^1$, $R^2$ and $R^3$ are each different from each other; and Y is a lone pair of electrons or a Lewis acid having vacant p orbitals for forming a stable complex with the ring nitrogen;

(b) separating the diastereomers of formula (II) to give a substantially pure diastereomer of said derivative; and

(c) hydrolysing the substantially pure diastereomer of said derivative so produced to give the corresponding substantially pure enantiomer of formula (I); and, optionally, converting the enantiomer of formula (I) to a reactive derivative thereof.

Preferably, the process is carried out wherein the diastereomer of formula (II) is in either exo- or endo-form.

Suitable alkyl groups for R, $R^1$, $R^2$ and $R^3$ are for example $C_{1-6}$ alkyl, such as methyl, ethyl, propyl and butyl. Suitable aryl groups include phenyl and naphthyl. Suitable aralkyl groups include benzyl.

Preferably the group R represents H.

Preferably, in formula (II), the groups $R^1$, $R^2$ and $R^3$ are of different physical sizes from each other to enable easier separation. Particularly preferred is where $R^1$ is H, $R^2$ is methyl and $R^2$ is phenyl or naphthyl.

The Lewis acid, when present, (Y in formula (II)) may be any one having p vacant orbitals capable of forming a stable complex with the azabicycle such as borane, or substituted boranes such as those substituted with alkyl or halo (such as F) groups, or dimers thereof. Preferably, Y is $BH_3$ (borane).

The diastereomers of formula II are novel compounds and form a further aspect of this invention.

A favoured compound of formula (II) is wherein R and $R^1$ are H, $R^2$ is methyl and $R^3$ is phenyl or naphthyl, preferably phenyl. Particularly favoured is where x is zero and y is 1 (i.e. a substituted 1-azabicyclo-[2.2.1]heptane).

Separation of the diastereomers of formula (II) to give a substantially pure enantiomer thereof (step B) may be carried out by standard methods such as chromatography. When Y is a lone pair, separation may be carried out on alumina, but the separation is preferably carried out when Y is a Lewis acid by column chromatography.

To convert the substantially pure diastereomer of formula (II) to the desired enantiomer of formula (I) (step (c)), any standard method for converting amides to acids and decomplexing Lewis acid complexes may be used such as hydrolysis under acid or alkali conditions. Preferably, hydrolysis is done in acid conditions under reflux such as refluxing with concentrated hydrochloric acid.

Once the substantially pure enantiomer of formula (I) is obtained, it may optionally be converted by standard conditions to a derivative thereof suitable for use as an intermediate in the synthesis of, inter alia, the muscarinic agonists mentioned above. Preferably, the enantiomer of formula (I) is esterified by standard methods to form, for example, alkyl or aryl esters thereof such as the methyl or benzyl esters thereof. Preferred esterifying agents are ones in which the enantiomer of formula (I) is soluble, such as methanol (to form the methyl ester). Esterification is best done in the presence of a mineral acid such as hydrochloric or sulphonic acids (more preferably HCl), optionally in the presence of, for example, methyl orthoformate, or by using diazomethane.

Alternatively, the carboxy group (-COOH) of the enantiomer of formula (I) may be converted, for example, to cyano (-CN), amido ($-CONH_2$) or $-C=NOH(NH_2)$.

Thus, the present invention also provides a method for resolving the enantiomers of other intermediates in the preparation of, inter alia, the muscarinic agonists mentioned above, which are derivatives at the carbon atom $\beta$ to the ring nitrogen of the azabicyclic enantiomers of formula (I).

To prepare diastereomers of formula (II) starting from an unresolved mixture of enantiomers of formula

(I) or a reactive derivative thereof (the racemate) (V), it is possible either to prepare a chiral amide derivative thereof and then, optionally, complex this with the Lewis acid (Y) [scheme A] or vice versa, (i.e. to prepare the Lewis acid complex of the racemate and then form a chiral amide of the complex) [scheme B].

Schemes A and B are illustrative only and show the case wherein, in formula (I), x = o, y = 1, Y = $BH_3$ and the reactive derivative is a methyl ester; and in formula (II), R and $R^1$ = H, $R^2$ = methyl and $R^3$ = phenyl.

In scheme B, the Lewis acid complex is prepared from an ester derivative of the racemate such as the methyl ester (V) [step BA]. Otherwise, in schemes A and B, the complexing is undertaken in like manner by adding the Lewis acid in an aprotic solvent at a reduced temperature. A typical solvent is tetrahydrofuran. The temperature is preferably in the range of from 0° to -80° C such as around -70° C.

Scheme A

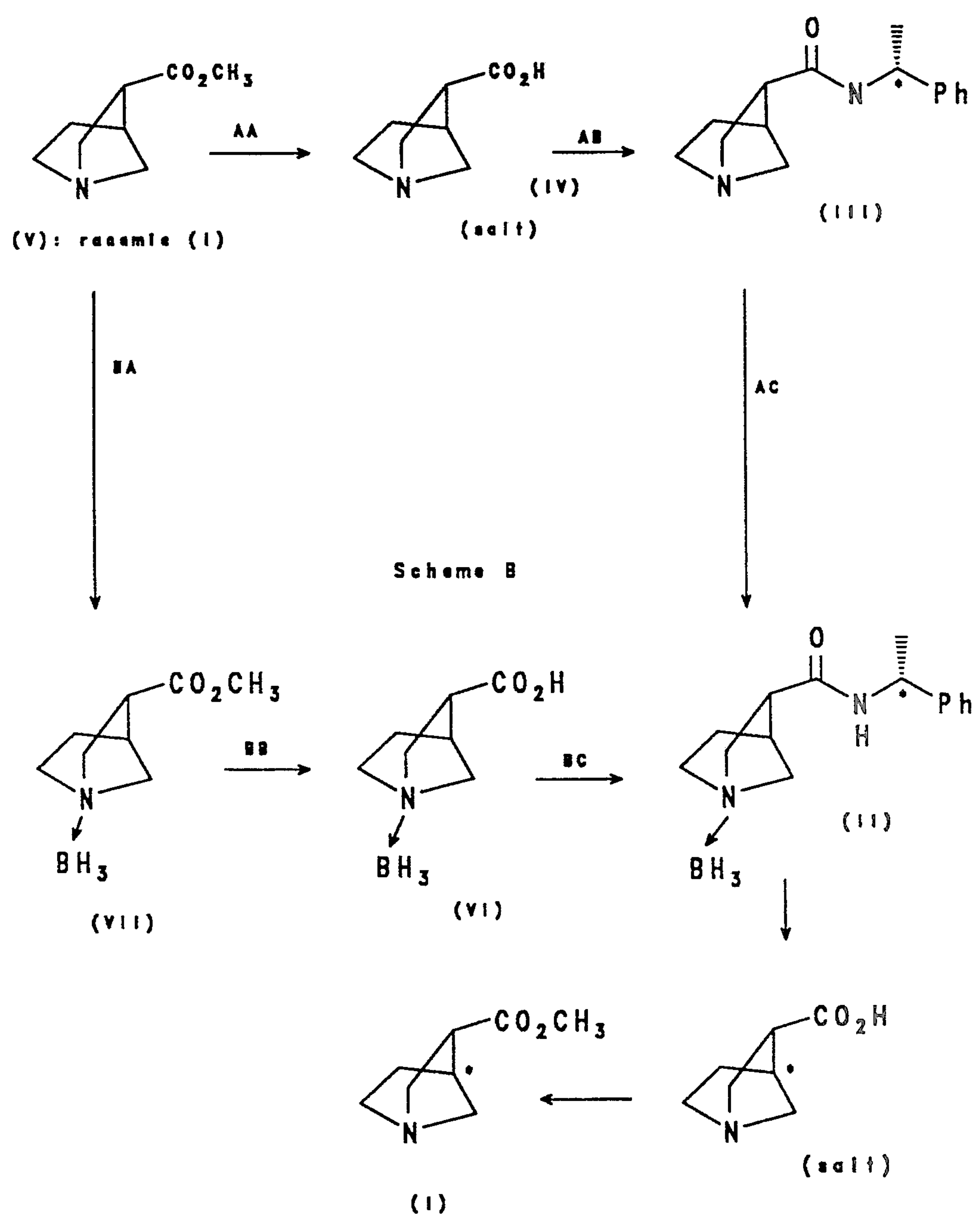


(V): racemic (I)

(salt)

(IV)

(III)

Scheme B

(VII)

(VI)

(II)

(salt)

(I)

In schemes A and B, the addition of the chiral amine takes place on a carboxy group and therefore any esters formed at previous reaction steps must be hydrolysed. In the case of scheme A, a starting racemic ester (V) can be hydrolysed under standard conditions with an aqueous volatile mineral acid such as

hydrochloric acid (the hydrochloride salt is formed under these conditions) [step AA]. In the case of scheme B, wherein the Lewis acid complex of the racemate (VII) has been formed, standard basic conditions are used such as an alkali or alkaline earth metal hydroxide or carbonate (eg. NaOH, LiOH, $Ba(OH)_2$, $Na_2CO_3$) in an alcoholic solvent (eg. methanol) at room temperature [step BB].

Finally to prepare the chiral amides (III) and (II), the intermediates (IV) and (VI) so far prepared are reacted with a chiral amine of formula (VIII)

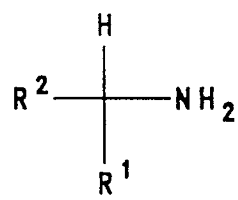

(VIII)

in either S or R form. Preferably, either (R)-(+)-or (S)-(-)-benzylamine is used.

For scheme A, the intermediate (IV) is refluxed with thionyl chloride or oxalylchloride and then the chiral amine (VIII) added at reduced temperature in an inert solvent [step AB]. A suitable temperature is in the range of from 0° C to room temperature. A suitable solvent is one which is chlorinated in the case where the intermediate (IV) is in the form of an HCl salt, such as dichloromethane.

For scheme B, the intermediate (VI) is reacted with a mixed carbonic anhydride at reduced temperature, for example, in the range of from -30° to -10° C (preferably at about -20° C) for a period of about 10 minutes or less [step BC]. The anhydride is prepared *in situ* by standard methods, such as from a haloformate in the presence of a weak base in an aprotic solvent. Suitable haloformates are chloroformates such as lower alkyl chloroformates, for example iso-butyl chloroformate or ethyl chloroformate. The base may be selected from tertiary amines such as triethyl amine or N-methylmorpholine, or diisopropyl ethylamine. The solvent may be dimethylformamide or, preferably, tetrahydrofuran. A preferred method is to use iso-butyl chloroformate in the presence of triethylamine in tetrahydrofuran. The chiral amine (VIII) is then added at the same (ie -30° to -10° C) or a lower temperature which temperature is then allowed to rise to from 0° C to room temperature.

Then, the diastereomers of formula (III) may be separated as described in step (b) above and then the resulting diastereomer hydrolysed as described in step (c) above (where Y is a lone pair) or, preferably, the Lewis acid is added as shown in the schemes, with separation of the diastereomers of formula (II) following.

Preferably, the diastereomer of formula (II) is prepared *via* scheme B.

The present invention will now be illustrated by the following examples, although other ways of putting it into effect will be clear from the foregoing description to the person skilled in the art.

## EXAMPLE 1

### (-) Exo-Methyl 1-Azabicyclo[2.2.1]heptane-3-carboxylate Hydrogen Oxalate

#### a) Exo Methyl 1-Azabicyclo[2.2.1]heptane-3-carboxylate Borane complex

Borane-tetrahydrofuran complex (242.5mL of a 1M tetrahydrofuran solution, 0.24mol) was added dropwise to a stirred solution of (+) exo methyl-1-azabicyclo [2.2.1]heptane-3-carboxylate (15.0g, 0.097mol, *J. Chem. Soc. Chem. Commun.*, **1988**, 1618) in anhydrous tetrahydrofuran (200mL) at -78° C under a nitrogen atmosphere. After a further 0.5h at -78° C water (20mL) was added dropwise and the mixture stirred vigorously whilst warming to room temperature. The majority of the tetrahydrofuran was removed by distillation and further water was added. The aqueous was extracted using dichloromethane (twice) and the combined organics were dried (sodium sulphate) then evaporated to give a pale yellow oil (16.5g). This oil

was purified by column chromatography on silica using ethyl acetate/n-hexane (1:2) to provide the title compound as a colourless solid (12.0g, 73%), mp 35-36 °C. $R_f$ 0.35 in ethyl acetate/n-hexane (1:1) on silica plates; m/z 168 for $(M-H)^+$; $n_{max}$ (film) 2450-2250cm$^{-1}$ (B-H), 1735cm$^{-1}$ (C=O); 'H NMR (360MHz, $CDCl_3$) δ 1.60-1.68 (1H, m, 5-CH); 1.98-2.08 (1H, m, 5-CH) overlapped with 1.20-2.10 (3H, broad resonance, $BH_3$); 2.66-2.73 (2H, m), 2.84-2.92 (1H, m), (1H, m, 2-$CH_2$, 3-CH, 4-CH, 6-$CH_2$ and 7-$CH_2$), 3.73 (3H,s, $CO_2CH_3$). (Found: C, 56.86; H, 9.44; N, 8.22. $C_8H_{16}NO_2B$ requires C, 56.84; H, 9.54; N, 8.29%).

b) Exo 1-Azabicyclo[2.2.1]heptane-3-carboxylic acid Borane complex

A solution of sodium hydroxide (3.08g, 77mmol) in water (40mL) was added dropwise to a stirred, cooled (0 °C) solution of the foregoing ester (10g, 59mmol) in methanol (80mL). The mixture was allowed to warm to room temperature then stirred for 4 hours. The methanol was evaporated and further water (80mL) was added. The aqueous was treated with citric acid (7.77g, 40mmol) then extracted with dichloromethane (three times). The combined organics were dried (sodium sulphate) then evaporated *in vacuo* to give the acid as a colourless solid (7.05g, 77%), mp 82-83 °C (diethyl ether/petroleum ether (40-60)). $R_f$ 0.25 in dichloromethane/methanol (19:1) on silica plates; m/z 154 for $(M-H)^+$; $\nu_{max}$ (nujol) 3300-2500cm$^{-1}$ (OH), 2400-2250cm$^{-1}$ (B-H), 1700cm$^{-1}$ (C=O); 'H NMR (360MHz, $CDCl_3$) δ 1.62-1.71 (1H, m, 5-$CH_2$), 2.02-2.12 (1H, m, 5-$CH_2$) overlapped with 1.20-2.20 (3H, broad resonance, $BH_3$), 2.72-2.77 (2H, m), 2.86-3.21 (5H, m) and 3.43 (1H, ddd, J = 3,6 and 13Hz, 2-$CH_2$, 3-CH, 4-CH, 6-$CH_2$ and 7-$CH_2$). (Found: C, 54.25; H, 9.03; N, 9.08. $C_7H_{14}NO_2B$ requires C, 54.24; H, 9.10; N, 9.04%).

c) 1R-Phenylethyl 1-Azabicyclo[2.2.1]heptane-3-carboxamide Borane complex

Triethylamine (8.9mL, 64mmol) was added to a stirred, cooled (-20 °C) solution of the foregoing acid (8.0g, 52mmol) in anhydrous dichloromethane (100mL) under a nitrogen atmosphere. Isobutylchloroformate (7.5mL, 57mmol) was added dropwise keeping the temperature beloδ -20 °C. After 15 minutes (R)-(+)-a-methylbenzylamine (6.9g, 57mmol) was added, again keeping the temperature at -20 °C. After a further 1h at -20 °C water (60mL) was added and the mixture stirred whilst warming to room temperature. The organic layer was separated and the aqueous re-extracted with dichloromethane. The combined organics were dried (sodium sulphate) then evaporated to give a solid. Exhaustive chromatography on silica using ethyl acetate/petroleum ether (40-60) (1:1) provided diastereomer 1 as a colourless crystalline solid (3.5g, 26%) and diastereomer 2 as a colourless crystalline solid (2.2g, 17%).

Diastereomer 1, mp 151 °C. HPLC: 99.4% on an acetylated β-cyclodextrin column, 50% methanol in water; m/z 257 for $(M-H)^+$; HRMS, found: $(M-H)^+$ 257.1836, $C_{15}H_{22}BN_2O$ requires (M-H) 257.1825; $\nu_{max}$ - (nujol) 3340cm$^{-1}$ (N-H), 2260, 2310 and 2350cm$^{-1}$ (B-H), 1645cm$^{-1}$ (C=0); $[a]_D^{22°C}$ + 64.0° (c=0.5, $CH_2Cl_2$); 'H NMR (360MHz, $CDCl_3$) δ 1.48 (3H, d, J = 7Hz, $CHCH_3$), 1.52-1.70 (1H, m, 5-CH), 1.91-2.02 (1H, m, 5-CH) overlapped with 1.20-2.24 (3H, broad resonance, $BH_3$), 2.40 (1H, dd, J = 6.9 and 7Hz, 3-CH), 2.64 (1H, d, J = 10Hz), 2.76 (1H, d, J = 4Hz, 4-CH), 2.79-2.90 (1H, m), 2.98-3.08 (1H, m), 3.08-3.20 (1H, m), 3.24 (1H, d, J = 9Hz), 3.42-3.48 (1H, m), 5.09 (1H, quintet, J = 7Hz, $CHCH_3$), 5.58-5.67 (1H, m, NH), 7.19-7.42 (5H, m, $C_6H_5$). (Found: C, 69.39; H, 8.87; N, 10.80. $C_{15}H_{23}BN_2O$ requires C, 69.78; H, 8.98; N, 10.85%).

Diastereomer 2, mp 176-178 °C. HPLC: 98% on an acetylated β-cyclodextrin column, 50% methanol in water; m/z 257 $(M-H)^+$; HRMS, found: $(M-H)^+$ 257.1833, $C_{15}H_{22}BN_2O$ requires (M-H) 257.1825; $\nu_{max}$ (nujol) 3200-3380cm$^{-1}$ (NH), 2260, 2310 and 2340cm$^{-1}$ ($BH_3$), 1640cm$^{-1}$ (C=0); $[a]_D^{22°C}$ + 91.15° (c=0.5, $CH_2Cl_2$); 'H NMR (360MHz, $CDCl_3$) δ 1.50 (3H, d, J = 7Hz, $CHCH_3$), 1.54-1.62 (1H, m, 5-CH), 1.94-2.08 (1H, m, 5-CH), overlapped with 1.36-2.18 (3H, broad resonance, $BH_3$), 2.38 (1H, dd, J = 6.6 and 7.3Hz, 3-CH), 2.64 (1H, d, J = 10Hz), 2.78-2.89 (1H, m) overlapped with 2.84 (1H, d, J = 4Hz, 4-CH), 2.93-3.00 (1H, m), 3.07-3.18 (1H, m), 3.26 (1H, d, J = 9.6Hz), 3.38-3.44 (1H, m), 5.09 (1H, quintet, J = 7Hz, $CHCH_3$), 5.60-5.74 (1H, m, NH), 7.24-7.45 (5H, m, $C_6H_5$). (Found: C, 69.77; H, 8.91; N, 10.79. $C_{15}H_{23}BN_2O$ requires C, 69.78; H, 8.98; N, 10.85%).

d) (-) Exo Methyl 1-Azabicyclo[2.2.1]heptane-3-carboxylate Hydrogen Oxalate

The foregoing amide diastereomer 1 (3.3g, 12.8mmol, $[a]_D^{22°C}$ + 64.0°) was heated at reflux in concentrated hydrochloric acid (30mL) for 20 hours. The solution was evaporated to dryness, the residue

dissolved in aqueous sodium hydroxide solution (3.5mol equivalents), washed with diethyl ether then re-acidified with concentrated hydrochlorio acid. The mixture was evaporated to dryness and the residue dried under vacuum over phosphorus pentoxide to yield a solid (7.3g).

Thionyl chloride (2.79mL, 38.4mmol) was added to methanol (30mL) with stirring at -20° C. The crude acid (7.3g, prepared above) was added and the solution allowed to warm to room temperature then stirred for 20 hours. The methanol was evaporated in vacuo and the colourless residue dissolved in water. Dichloromethane was added and the aqueous was basified to pH 10 with potassium carbonate. The organic layer was separated and the aqueous was re-extracted with dichloromethane (four times). The combined organics were dried (sodium sulphate) then evaporated to dryness in vacuo and the residue purified by column chromatography on silica using 6% methanol in dichloromethane to afford the free base as an oil (1.02g, 51%). The hydrogen oxalate had mp 114-115° C (methanol). $R_f$ 0.26 in dichloromethane/methanol (9:1) on silica; m/z 155 for $M^+$ free base, HRMS, found: $M^+$ 155.0984, $C_8H_{13}NO_2$ requires M 155.0946; $[a]_D^{22°C}$ -3.2° (c=0.5, MeOH); 'H NMR (360MHz, $D_2O$) δ 1.80-1.89 (1H, m, 5-CH), 2.11-2.22 (1H, m, 5-CH), 3.07 (1H, dd, J = 5.4 and 8.6Hz, 3- CH), 3.15-3.34 (4H, m), 3.38-3.52 (2H, m) and 3.70 (1H, ddd, J = 3,5.5 and 12Hz, 2-$CH_2$, 4-CH, 6-$CH_2$ and 7-$CH_2$), 3.76 (3H, s, $CO_2$ $CH_3$). (Found: C, 48.79; H, 6.15; N, 5.72. $C_8H_{13}NO_2.C_2H_2O_4$ requires C, 48.98; H, 6.17; N, 5.71%).

## EXAMPLE 2

### (+) Exo Methyl 1-Azabicyclo[2.2.1]heptane-3-carboxylate Hydrogen Oxalate

This was prepared from 1R-phenylethyl 1-azabicyclo [2.2.1]heptane-3-carboxamide borane complex, diastereomer 2 (1.85g, 7.2mmol, $[a]_D^{22°C}$ + 91.15°, Example 1(c)) as fully described in Example 1(d), mp 115° C (methanol). $R_f$ 0.26 in dichloromethane/methanol (9:1) on silica; m/z 155 for $M^+$ of free base; HRMS, found: $M^+$ 155.0969, $C_8H_{13}NO_2$ requires M 155.0946; $[a]_D^{22°C}$ + 3.0° (c=0.5, MeOH). (Found: C, 48.85; H, 6.16; N, 5.72. $C_8H_{13}NO_2.C_2H_2O_4$ requires C, 48.98; H, 6.17; N, 5.71%).

## EXAMPLE 3

### 1R-Phenylethyl 1-azabicyclo[2.2.1]heptane-3-carboxamide borane complex

(Method B)

a) 1R-Phenylethyl 1-Azabicyclo[2.2.1]heptane-3- carboxamide

A solution of (+) Exo methyl 1-azabicyclo[2.2.1] heptane-3-carboxylate (3.0g, 19.4mmol) in concentrated hydrochloric acid (20ml) was heated under reflux for 18h, cooled and evaporated to dryness to yield exo 1-azabicyclo[2.2.1]heptane-3-carboxylic acid hydrochloride (3.44g, 100%). A portion of this material (1.0g, 5.63mmol) was suspended in thionyl chloride (20mL) and heated under reflux for 2h. The cooled solution was evaporated and toluene (2 x 20ml) evaporated from the residue. This material was dissolved in dichloromethane (30mL), cooled in ice and 1R-phenylethylamine (1.5mL, 11.3mmol) added with stirring followed by triethylamine (1.5ml, 11.3mmol). The cooling bath was removed and the mixture stirred overnight. Potassium carbonate solution (2M, 20mL) was added and the product extracted with dichloromethane (4 x 20ml), dried over magnesium sulphate and evaporated. The diastereomeric amides could be separated by column chromatography on alumina in dichloromethane/methanol (99:1) to give diasteromer 1, (208mg, 15%), mixed fractions (95mg) and diastereomer 2 (209mg, 15%). Diastereomer 1 was free of diastereomer 2, but was contaminated with unreacted phenylethylamine. m/z 244 ($M^+$); 'H NMR (360MHz, $CDCl_3$) δ 1.12-1.20 (1H, m, 5-CH), 1.49 (3H, d, J = 7Hz, $CHCH_3$), 1.58-1.68 (1H, m, 5-CH), 2.03 (1H, dd, J = 6.8Hz and 7Hz, 3-CH), 2.36 (1H, d, J = 10Hz, 7-CH), 2.43-2.34 (1H, m), 3.72-3.78 (2H, m), 3.82-3.94 (2H, m) and 3.04-3.12 (1H, m, 2-$CH_2$, 4-CH, 6-$CH_2$ and 7-CH), 5.09 (1H, quintet, $CHCH_3$), 5.81

(1H, broad, NH), 7.16-7.38 (5H, m, $C_6H_5$).

Diastereomer 2 was pure by NMR. m/z 244 ($M^+$); 'H NMR (360MHz, $CDCl_3$) δ 1.08-1.19 (1H, m, 5-CH), 1.47 (3H, d, J = 7Hz, $CHCH_3$), 1.46-1.66 (1H, m, 5-CH), 2.03 (1H, t, J = 7Hz, 3-CH), 2.32 (1H, d, J = 10Hz, 7-CH), 2.43-2.55 (1H, m, 6-CH), 2.68 (1H, d, 3 = 4Hz, 4-CH), 2.70-2.84 (1H, m), 2.84-2.96 (2H, m) and 3.08-3.16 (1H, m, 2-$CH_2$, 6-CH and 7-CH), 5.09 (1H, quintet, J = 7Hz, $CHCH_3$), 5.83 (1H, broad, NH), 7.16-7.40 (5H, m, $C_6H_5$).

b) 1R-Phenethyl-1-azabicyclo[2.2.1]heptane-3-carboxamide borane complex

Although the amide diastereomers could be separated as described above, unreacted amine could not be removed conveniently. It was preferable to convert to the borane complex by treating the crude amide from a similar preparation (129mg, 0.53mmol) in tetrahydrofuran (10ml) with borane-tetrahydrofuran complex (2.0ml of 1M solution) at -78° C and working up exactly as described in Example 1a. Filtration through a plug of silica in dichloromethane/ethyl acetate (95:5) and evaporation of the eluate yielded the diastereomeric amide borane complexes (53mg, 39%), now free of phenylethylamine. The isomers were separated as described in Example 1c and were identical in all respects.

## Claims

1. A process for preparing a substantially pure enantiomer of a 1-azabicyclo[2.2.1]heptane, 1-azabicyclo[3.2.1] octane or quinuclidine, substituted on the carbon atom β to the ring nitrogen by a carboxy group, which process comprises:

(a) synthesising diastereomeric derivatives thereof wherein the hydroxy group of the carboxy is replaced by a residue of a chiral amine or α-amino acid;

(b) separating said diastereomeric derivatives thereof to give a substantially pure diastereomer of said derivative; and

(c) hydrolysing the substantially pure diastereomer of said derivative so produced to give the corresponding substantially pure enantiomer.

2. A process for preparing substantially pure enantiomers of formula (I)

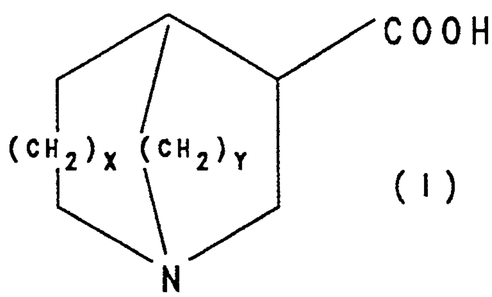

(I)

wherein x is 0 or 1, y is 1 or 2 and x plus y is less than 3, in exo-, endo- or a mixture of exo- and endo-forms; or a reactive derivative thereof, which process comprises

(a) synthesising diastereomers of formula (II):

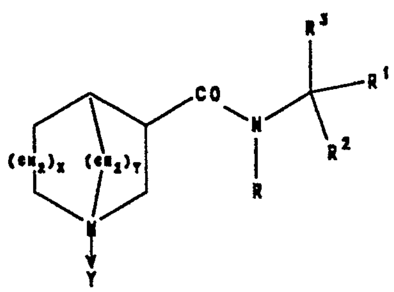

(II)

wherein R, $R^1$, $R^2$ and $R^3$ are each selected from H, alkyl, aryl and aralkyl, and $R^1$ is additionally selected from a carboxylic acid derivative, provided that $R^1$, $R^2$ and $R^3$ are each different from each other; and Y is a lone pair of electrons or a Lewis acid having vacant p orbitals for forming a stable complex with the ring nitrogen;

(b) separating the diastereomers of formula (II) to give a substantially pure diastereomer of said derivative; and

(c) hydrolysing the substantially pure diastereomer of said derivative so produced to give the corresponding substantially pure enantiomer of formula (I); and, optionally, converting the enantiomer of formula (I) to a reactive derivative thereof.

3. A process as claimed in claim 2 wherein $R^1$ is hydrogen, $R^2$ is methyl and $R^3$ is phenyl or naphthyl.

4. A process as claimed in claim 1 or claim 2 wherein Y represents $BH_3$.

5. A process as claimed in any one of claims 2 to 4 wherein R represents hydrogen.

6. A process as claimed in any one of claims 2 to 5 wherein x is zero and y is 1.

7. A compound of formula (II), as defined in claim 2.

8. A compound as claimed in claim 7 wherein R and $R^1$ are H, $R^2$ is methyl and $R^3$ is phenyl.

9. A compound as claimed in claim 7 or claim 8 wherein Y is $BH_3$.

10. A compound as claimed in any one of claims 7 to 9 wherein x is zero and y is 1.